# EUROPÄISCHE PATENTSCHRIFT

(11) **EP 1 268 770 B1**
(45) Veröffentlichungstag und Bekanntmachung des Hinweises auf die Patenterteilung: **13.10.2004**
(21) Anmeldenummer: 02722236.3
(22) Anmeldetag: 18.03.2002
(51) Int. Cl.: C12N 15/10, B01D 15/08, B01D 71/00, C12P 19/34

(54) **VERFAHREN ZUR ISOLIERUNG VON PLASMIDEN AUS SUSPENDIERTEN BAKTERIENZELLEN**
METHOD FOR ISOLATING PLASMIDS FROM SUSPENDED BACTERIAL CELLS
PROCEDE D'ISOLATION DE PLASMIDES A PARTIR DE CELLULES BACTERIENNES EN SUSPENSION

(30) Priorität: 21.03.2001 DE 10113815
(43) Veröffentlichungstag der Anmeldung: 02.01.2003
(73) Patentinhaber: EPPENDORF AG, 22339 Hamburg (DE)
(72) Erfinder: KOLZAU, Thomas, Dr., 22399 Hamburg (DE); PLÜSTER, Wilhelm, Dr., Boulder, CO 80304 (DE)
(74) Vertreter: Emmel, Thomas, Dipl.-Biol., Dr.
(86) Internationale Anmeldenummer: PCT/EP2002/002964
(87) Internationale Veröffentlichungsnummer: WO 2002/074954

(56) Entgegenhaltungen:
- EP-A- 0 814 156
- WO-A-00/40697
- DE-A- 4 321 904
- DE-A- 10 006 591
- US-A- 6 020 186
- WICKS I P ET AL: "BACTERIAL LIPOPOLYSACCHARIDE COPURIFIES WITH PLASMID DNA: IMPLICATIONS FOR ANIMAL MODELS AND HUMAN GENE THERAPY" HUMAN GENE THERAPY, XX, XX, Bd. 6, Nr. 3, 1. März 1995 (1995-03-01), Seiten 317-323, XP000676948 ISSN: 1043-0342

## Beschreibung

Die Erfindung bezieht sich auf Verfahren, bei denen Bakterienzellen lysiert und dann die nach der Lyse freigesetzten Plasmide unter Verwendung einer Filtermatrix isoliert werden.

Derartige Verfahren werden routinemäßig mit unterschiedlichen Typen von Zellen durchgeführt.

Ein häufiger Anwendungsfall ist z.B. die Isolierung von Plasmiden aus Bakterienzellen. Bei einem üblichen Verfahren werden die Bakterien pelletiert, und das Pellet wird dann durch Zugabe eines ersten Puffers resuspendiert. Dann wird zu der Suspension ein weiterer Puffer hinzugegeben, der die Lyse der Zellen bewirkt. In aller Regel ist dieser Puffer stark alkalisch und enthält hochwirksame Detergentien, z.B Natriumdodecylsulfat (SDS). Schließlich wird noch ein weiterer Puffer hinzugegeben, mit dem die Suspension wieder neutralisiert wird. Diese dreistufige Pufferzugabe wird auch als alkalische Lyse bezeichnet.

In einem weiteren Schritt wird dann die Suspension z.B. filtriert. Man erhält ein klares Filtrat, das die Plasmide enthält. Dem Filtrat wird ein Bindungspuffer zu- . gesetzt, der chaotrope Reagenzien und Silikapartikel enthält. Die in dem Bindungspuffer enthaltenen chaotropen Reagenzien bewirken, daß die Plasmide selektiv an den Silikapartikeln binden.

Die Plasmide können nach Waschen der Silikapartikel durch einen entsprechenden Elutionspuffer von den Silikapartikeln eluiert werden und liegen dann gereinigt vor.

Die beschriebene Aufarbeitung der Zellen bis zur Bindung der Plasmide an der Silikamatrix erfordert den Einsatz mehrerer unterschiedlicher Puffer sowie die Durchführung einer ganzen Reihe von unterschiedlichen Zentrifugations/Filtrier-, Dekantier- und Pipettierschritten. Dies ist zeit- und kostenaufwendig und erschwert darüber hinaus eine Automatisierung.

Aus der WO-A-0040697 ist ein Verfahren bekannt, bei dem Zellen bei Kontakt mit einer Filtermatrix lysieren, und die längeren Nukleinsäuren an die Matrix gebunden werden. Die Plasmide passieren bei diesem Verfahren zusammen mit RNA, Proteinen und anderer Biomolekülen die Matrix und müssen zum Erhalt reiner Plasmide durch nachgeschaltete Reinigungsschritte aus dem Filtrat isoliert werden.

Aus dem US-Patent 6020186 ist ein Verfahren bekannt, bei dem die Zellen an einer Filtermatrix gebunden und dann lysiert werden. Die freigesetzte Plasmid-DNA wird an Ionen-Austauscher-Partikel gebunden und wird unter Hochsalzbedingungen oder unter Verwendung von alkalischen Puffern eluiert.

DE-A-4321904 schließlich offenbart ein Verfahren zur Isolierung von Plasmiden aus Bakterienzellen, bei dem pelletierte Zellen suspendiert und mittels alkalischer Lyse aufgeschlossen werden, das Lysat mit einem Träger in Kontakt gebracht wird und anschließend die Plasmide von dem Träger selektiv eluiert werden. Optional ist vorgesehen, daß eine Substanz zugegeben werden kann, die die Bindungseigenschaften von Nukleinsäuren an dem eingesetzten Träger verbessert, wie z.B. PEG.

Aufgabe der Erfindung ist es demgegenüber, ein schnelleres und vereinfachtes Verfahren zu Isolierung von Plasmiden aus Bakterienzellen zu schaffen.

Gelöst wird die Aufgabe mit einem Verfahren, das die kennzeichnenden Merkmale des Anspruchs 1 aufweist.

Wie beim Stand der Technik werden die Zellen zunächst suspendiert. Der hierzu erfindungsgemäß eingesetzte Puffer enthält mind. eine lysierend wirkende Substanz, die die aufzuarbeitenden Zellen angreift und mindestens destabilisiert. Bevorzugt ist der Puffer so zusammengesetzt, daß er die Zellen schädigt, ohne daß der Zellinhalt austritt. Von der Erfindung abgedeckt werden soll aber auch die Möglichkeit, daß bei Vermischung der Zellen mit dem Puffer eine vollständige Lyse unter nicht denaturierenden oder nur geringfügig denaturierenden Bedingungen der Zellen eintritt.

Es versteht sich, daß obige Angaben statistisch zu betrachten sind. Bei beiden beschriebenen Möglichkeiten wird zu einem gewissen Prozentsatz auch jeweils der andere nicht vorrangig gewünschte Effekt eintreten, d.h. bei der ersten Möglichkeit läßt sich nicht ausschließen, daß neben vorgeschädigten Zellen auch vollständig lysierte Zellen vorliegen und umgekehrt. Wenn im folgenden daher von lysierten bzw. vorgeschädigten Zellen etc. gesprochen wird, ist damit gemeint, daß mindestens der überwiegende Teil der Zellen in diesem Zustand vorliegt.

Der erfindungsgemäß eingesetzte Puffer enthält weiterhin eine oder mehrere Substanzen, die eine Konformationsänderung der Plasmide bewirken dergestalt, daß diese sich in bzw. an der Filtermatrix besser oder überhaupt zurückhalten lassen.

Ein bevorzugt eingesetzter erfindungsgemäßer Puffer für die Plasmidisolierung aus Bakterienzellen kann z.B. eine hyper- oder hypoosmotische Salzkonzentration aufweisen und kann darüberhinaus Lysozym und Fällungsmittel, bevorzugt Polyethylenglykol (PEG) aber auch Polyvinylpyrolidon (PVP), Ethanol oder Isopropanol oder chaotrope Salze wie z.B. Guanidinisothiocyanat oder Harnstoff enhalten.

Die Anwesenheit von Lysozym in Verbindung mit der eingestellten Salzkonzentration führt zu einer Schädigung der Bakterienzellen ggf. zur Lyse. Das PEG dagegen bewirkt, daß die nach Lyse der Zellen austretenden Plasmide eine Kondensation erfahren und dann in kondensierter Form in den Poren einer üblichen Filtermatrix zurückgehalten werden können.

Unter Kondensation sollen durch äußere Umstände hervorgerufenen Konformationsänderungen verstanden werden, die Stauchungen des Plasmids bis hin zum unlöslichen Zustand umfassen. Die Stauchung des Plasmids geht einher mit abnehmender Flexibilität und zunehmender Komplexizität.

Der Begriff Suspension wird im Rahmen dieser Anmeldung im wesentlichen dann gebraucht, wenn noch intakte bzw. lediglich vorgeschädigte Zellen vorliegen. Sind die Zellen dagegen vollständig lysiert, dann wird von Zellfraktionen gesprochen.

Erfindungsgemäß ist für den Fall, daß die Zellen zunächst nur vorgeschädigt werden, weiterhin vorgesehen, daß die Suspension z.B. auf eine Filtermatrix zentrifugiert, bzw unter Vakuum durch eine Filtermatrix hindurchgesogen wird. Dabei werden die Zellen noch weitgehend intakt gegen die Filtermatrix gedrückt. Aufgrund ihrer Vorschädigung sind die Zellen sehr empfindlich gegenüber mechanischen Belastungen. Die bei Kontakt mit der Filtermatrix auftretenden Scherkräfte reichen daher in aller Regel aus, um die vorgeschädigten Zellen aufreißen lassen.

Die Zellen geben dann im Bereich der Filtermatrix ihren Zellinhalt frei, wobei wie oben angesprochen, die zu isolierenden Plasmide aufgrund des z.B. in dem Puffer enthaltenen PEG die gewünschte Konformationsänderung erfahren, aufgrund der sie selektiv an der Filtermatrix zurückgehalten werden können.

Ein wesentlicher Vorteil gegenüber aus dem Stand der Technik bekannten Verfahren ist, daß die erfindungsgemäße Lyse der Zellen unter nicht oder nur schwach denaturierenden Bedingungen durchgeführt werden kann. Die freigesetzten Zellinhaltsstoffe verbleiben dadurch nach der Lyse in Lösung. Die Lösung mit den Zellinhaltsstoffen kann insgesamt ohne zwischengeschaltete Reinigungs- und Renaturierungsschritte auf ein Filter zu selektiven Isolierung z.B. der Plasmide gegeben werden. Die Plasmide werden unter den erfindungsgemäß eingestellten Bedingungen im bzw. am Filter zurückgehalten und die anderen Inhaltstoffe passieren das Filter.

Eine Ausgestaltung sieht vor, daß die Zellen bereits nach Kontakt mit der zu Beginn der Aufarbeitung zugesetzten mind. einen lysierend wirkenden Substanz vollständig lysieren, wobei auch hier die Bedingungen nicht oder nur schwach denaturierend gewählt sind.

Nach der Lyse kann bei dieser Variante zunächst ein Schritt, z.B. Zentrifugationsschritt, vorgesehen werden, bei dem die Zelltrümmer entfernt werden. Die dann erhaltene Zellfraktion kann, falls nicht schon geschehen, mit der erfindungsgemäß vorgesehene mind. einen konformationsändernden Substanz vermischt werden, die selektiv die Löslichkeit der Plasmide herabsetzt, und dann durch die Filtermatrix bewegt werden.

Bei der eingangs erwähnten bislang in Routineverfahren eingesetzten sogenannten "alkalischen Lyse" erfolgt mit der Lyse eine Denaturierung der freigesetzten Inhaltsstoffe und die Plasmide müssen zur weiteren Aufarbeitung wieder renaturiert werden, wobei dann je nach Protokoll auch ein Zentrifugationsschritt und/oder Filtrationsschritt erforderlich ist, um die renaturierten Plasmide von den denaturierten Proteinen und weiteren Inhaltstoffen zu trennen.

Die EP 0473 575 betrifft eine Verbesserung der bekannten "alkalischen Lyse", bei der mit einem speziellen Puffer Lyse, Neutralisierung und Deproteinierung gleichzeitig erfolgen. Aber auch hier sind nachgeschaltete Reinigungsschritte erforderlich, bevor die eigentliche Reinigung (Isolierung) der DNA erfolgen kann.

Das erfindungsgemäße Verfahren ist gegenüber diesem bekannten Verfahren deutlich einfacher durchzuführen. Die Schritte der Renaturierung und anschließenden Zentrifugation können vollständig entfallen. Mit der Erfindung ist es also tatsächlich möglich, Lyse und anschließende Plasmidisolierung ohne zwischengeschaltete Zentrifugation oder andere Trennschritte durchzufüren.

Üblicherweise wird bei beiden beschriebenen Möglichkeiten die Filtermatrix dann gewaschen, um störende andere Zellkomponenten zu entfemen. Danach wird bevorzugt mit einem Puffer eluiert, der die Konformationsänderung der zu isolierenden Plasmide wieder rückgängig macht, worauf diese wieder von der Filtermatrix freigegeben werden.

Der Aufbau der Filtermatrix, bzw. der Durchmesser ihrer Poren sind erfindungsgemäß auf die zu isolierenden Zellkomponenten abgestimmt, also z.B. so gewählt, daß die Plasmide in der durch das PEG eingestellten Konformation in der Matrix zurückgehalten werden können.

Das erfindungsgemäße Verfahren bietet eine Reihe von Vorteilen. Wesentlicher Vorteil ist, daß von der (Re-)Suspendierung der aufzuarbeitenden Zellen bis zur Isolierung der Zellkomponenten an der Filtermatrix nur ein einziger Puffer eingesetzt werden kann. Als Arbeitsschritte sind hier lediglich die Herstellung der Suspension sowie ein Zentrifugationsschritt bzw. ein Filtrierschritt erforderlich.

Gegenüber bekannten Verfahren lassen sich mit dem erfindungsgemäßen Verfahren daher Zeit und Kosten einsparen. Eine Automatisierung des Verfahrens ist in einfacher Weise möglich.

Geeignete Pufferzusammensetzungen sowie geeignete Filtermatrizes sind in den weiter unten angeführten Beispielen angegeben.

Generell soll noch einmal hervorgehoben werden, daß alle Puffer geeignet sind, die Substanzen enthalten, die eine Schädigung der Zellen einschließlich ggf. Lyse und/oder eine Konformationsänderung der zu isolierenden Plasmide bewirken können.

Die Filtermatrix ist so gewählt, daß die bei Kontakt vorgeschädigterr Zellen mit der Matrix auftretenden mechanischen Kräfte eine vollständige Lyse der Zellen bewirken können und daß die zu isolierenden Zellkomponenten nach Konformationsänderung in oder an der Matrix zurückgehalten werden können.

Erstaunlicherweise hat sich herausgestellt, daß eine ganze Reihe von unterschiedlichen Filtermaterialien geeignet sind. Es scheint, daß die Zusammensetzung bzw. Oberflächenbeschaffenheit der Filtermatrix keinen maßgeblichen Einfluß auf die Umsetzung des erfindungsgemäßen Verfahrens hat. Mit anderen Worten, entsprechend vorgeschädigte Zellen reißen in den meisten Fällen unabhängig von den eingesetzten Filtern auf, wenn sie in Kontakt mit deren Oberfläche geraten.

Selbst wenn das erfinderische Verfahren relativ unabhängig von der eingesetzten Filtermatrix funktioniert, so lassen sich selbstverständlich über ihre Gestaltung die Ergebnisse optimieren.

Eine vorteilhafte Ausgestaltung in diesem Zusammenhang sieht vor, daß die Filtermatrix aus zwei Schichten aufgebaut ist. Die eine Schicht (obere Schicht) bildet die Oberfläche aus, die die vorgeschädigten Zellen kontaktieren, was dann zur vollständigen Lyse führt. Außerdem werden in der oberen Schicht nach Lyse der Zellen die größeren Zelltrümmer zurückgehalten.

Die interessierenden Plasmide sollen dagegen gezielt in der unteren Schicht der Filtermatrix aufgefangen werden.

In einer besonders bevorzugten Ausgestaltung können die Oberflächenstrukturen der unteren Filterschicht über das umgebende Puffermedium variabel einstellbar sein. So ist es z.B. denkbar, daß die Oberflächenstrukturen durch die gleichen Puffersubstanz(en) beeinflußt werden, die die gewünschte Konformationsänderung bei den zu isolierenden Plasmiden bewirken. Die Oberflächenstrukturen können z.B. Tentakel aufweisen, die in Abhängigkeit von dem eingesetzten Puffer z.B. den Querschnitt der Poren der Filtermatrix verändern. Liegen die Tentakel gestreckt vor, dann ist die Pore durchlässig. Sind die Tentakel dagegen gestaucht, so verengen sie den effektiven Querschnitt der Pore und hemmen den Durchtritt. In diesem Zusammenhang kann eine Membran mit funktionalisierter Oberfläche zum Einsatz kommen, wie sie z.B. in der DE 10106199.4 oder 10006590.2 beschrieben ist.

Werden die Pufferbedingungen zur Elution geändert, so kann dies gleichzeitig die Oberflächenstruktur der unteren Filterschicht und die Zellkomponenten beeinflussen, was wiederum ein leichteres Ablösen ermöglicht.

Die obere Filterschicht kann ebenfalls als z.B. flächige Membran vorgesehen werden. Denkbar ist aber auch, daß sie überhaupt erst während des Zentrifugieroder Filtrierschrittes gebildet wird. Hierzu werden in dem Puffer Beads vorgesehen, die z.B. bei der Zentrifugation zuerst sedimentieren und dann die obere Filterschicht, ggf. aber auch, falls keine zwei Schichten vorgesehen werden, die gesamte Filtermatrix ausbilden.

Zum besseren Verständnis soll im folgenden auf einige Beispiele eingegangen werden, die das erfindungsgemäße Verfahrens unter unterschiedlichen Bedingungen illustrieren.

### A Eingesetzte Puffer

### Puffer PI, (Prälytischer Puffer, hyperosmotisch):

50mM Tris pH8.0
10mM EDTA pH8.0
(1.5% Triton X-100)
(10-20% Saccarose)
50 ug/ml RNase A
0.4 g/l Lysozyme
pH 8.0

### Puffer II: (Prälytischer Puffer, hypo-osmotisch):

ddH₂O
(1% Triton X-100)
0.3g/l Lysozyme
1mM Tris
50 ug/ml RNase A
(0.4 g/l Lysozyme)

### Puffer PIII (Lysepuffer):

50-100 mM Tris
10-50 mM EDTA
(10-20 % Saccharose)
1-5% Triton X-100
100-200 ug/ml RNase A
0.8-8 g/l Lysozyme
(0.1-1 % SDS)
(Proteinase K)
pH 8.0-10.5

Die Puffer PI-PIII werden jeweils in Verbindung mit 12-20% PEG (alternativ PVP, Isopropanol, etc. ), 2.5 M NaCI im Verhältnis 1:1 eingesetzt. Zusätzlich können Beads unterschiedlicher Natur zugesetzt werden. Für diesen Fall werden die Bezeichnungen PI-PIII durch entsprechende Angabe der enthaltenen Beads ergänzt.

### B Filtermatrizes

### a. einschichtig:

PVDF 0.45 µm
PVDF 0,5 µm
Glasfaser/C
Glasfaser/F
Nylon 0,45 µm
Nylon 0,5 µm
PKB(B)
Nylon 0,45 mit chemisch funktionalisierter Oberfläche

### b. zweischichtig:

Glasfaser/F + MBP Support
Glasfaser/C 2-lagig
Nylon 0,2-1,2 µm + Cellulose

### c. Beads bzw. "Flüssigfilter"

Diverse Beads wurden auf ihre Eignung als obere Filterschicht nach Sedimentation getestet:
**B(1)** Davisil 200-425, Silica Porengröße 150 Angstroem, Partikelgröße 33-75 µm
**B(2)** SephadexG200, Dextran quervernetzt mit Epichlorhydrin, Partikelgröße 25-100 µm
**B(3)** Sephadex LH20, Partikelgröße 20-100
**B(4)** CelatomFW14, Cellulose
**B(5),** Celatom FW50, Cellulose
**B(6)** Celatom FW60, Cellulose
**B(7),** CelatomFW80, Cellulose
**B(8)** AFR25PDB-SO₃, CK10M/4PDB-SO0₃, Polystyrol quervernetzt mit Divinylbenzol, Ionenaustauscher, Partikelgröße ca. 20 µm
**B(9)** POROSR1-10, Polystyrol quervernetzt mit Divinylbenzol
**B(10)** POROSR1-20, Polystyrol quervemetzt mit Divinylbenzol
**B(11)** POROSR2-10, Polystyrol quervemetzt mit Divinybenzol
**B(12)** POROSR2-20, Polystyrol quervernetzt mit Divinylbenzol Kontrollen:
B(13) Glasbeads,
**B(14)** ohne Beads
**B(15)** leer
**B(16)** Perfect Prep Mini.

### Beispiel 1: Isolierung unterschiedlicher Plasmide aus unterschiedlichen E. Coli-Stämmen.

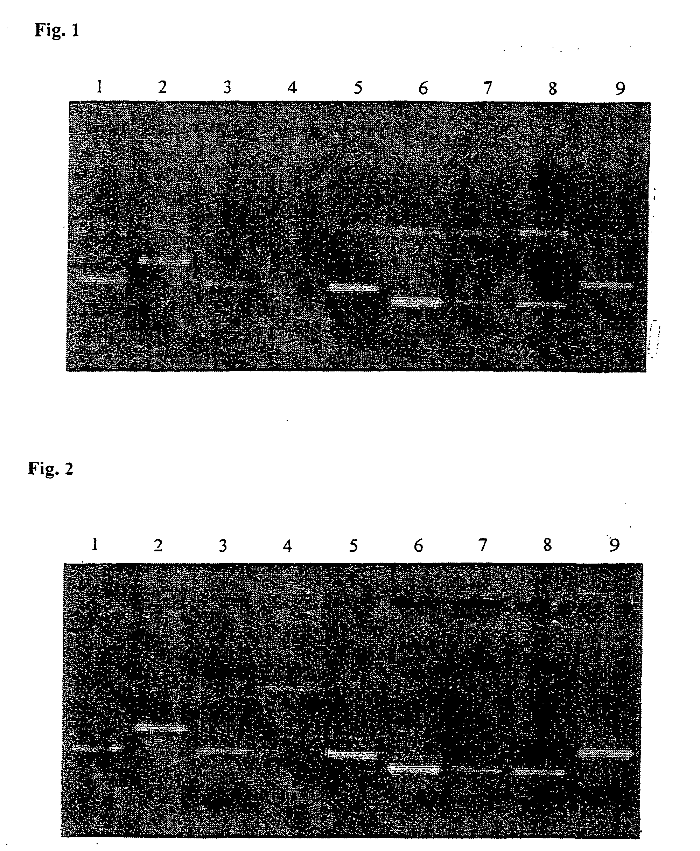

Die Zellen wurden jeweils in 1,2 LB Medium angezogen und dann pelletiert. Die Zell-Pellets wurden jeweils in 400 µl Puffer I + 30 µl Glasbeads aufgenommen und die erhaltene Suspension mittels Zentrifugation durch eine einschichtige Filtermatrix (Nylon 0,45) in Einzelsäulen gedrückt. Nach ethanolischem Waschen, wurde dann mit jeweils 30 µl TE-Puffer eluiert und die Eluate wurden gel-elektrophoretisch analysiert. (Fig 1.) zeigt Ergebnisse, die sich mit herkömmlichen Methoden erzielen lassen (Mini-Prep Eppendorf).

Man erkennt, daß das erfindungsgemäße Verfahren (Fig. 2) im wesentlichen dieselben Banden liefert, wie das herkömmliche Verfahren (Fig. 1), wobei allerdings das erfindungsgemäße Verfahren wesentlich weniger Verfahrensschritte beinhaltet. Die Zuordnung der Spuren zu den zu isolierenden Plasmiden ist wie folgt: Spur 1: pBluescript, (XL1Blue); Spur 2: pUC18 (XL1Blue); Spur 3: Q1.2 (XL1Blue); Spur 4: pBR322 (XL1Blue); Spur 5: GAPDH (HB101); Spur 6: pBluescript (DH10β); Spur 7: pBluescript (DH5α); Spur 8: pUC19 (JM109) und Spur 9: hsp70 (XL1Blue).

### Beispiel 2: Isolierung von Plasmiden unter Verwendung unterschiedlicher 2-schichtiger Filtermatrizes.

1,2 ml LB-Bakterienkulturen wurden pelletiert und danach mit jeweils 400µl Puffer I resuspendiert. Nach Übertragung in 96well Kavitäten wurde das Prälysat durch Zentrifugation durch eine 2 schichtige Matrix bestehend aus cellulose basierenden Material und Nylon der Porenweiten 0,2µm - 1,2 µm bewegt (Spuren 1,2 = 1,2µm; Spuren 3, 4 = 0,6µm; Spuren 5, 6 = 0,45µm; Spuren 7, 8 = 0,2µm; Spuren 9, 10 = EYMS 1,2µm; Spuren 11, 12 = MiniPrep).

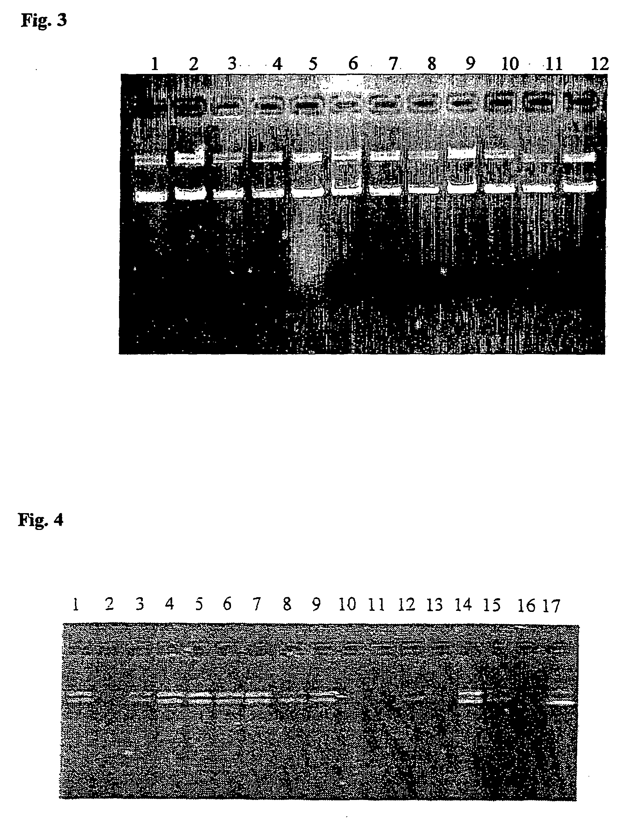

Nach einem ethanolischen Waschschritt, erfolgte die Elution mit 30µl TE Puffer. Fig. 3 zeigt die gelelektrophorethische Analyse der Präparationen im Vergleich zu einer herkömmlichen Plasmid Mini Präparation mit dem Perfect Prep Plasmid Mini Kit der Firma Eppendorf. Der Vergleich zur Kontrolle ergibt einen qualitativ und quantitativ übereinstimmenden Präparationserfolg, unter Reduzierung des Verfahrensaufwandes bei Durchführung der erfindungsgemäßen Methode.

### Beispiel 3: Isolierung von Plasmiden unter Verwendung unterschiedlicher Beads als Flüssigfilter.

1,2 ml LB-Bakterienkulturen wurden pelletiert, danach mit jeweils 400µl Puffer I versetzt und mit jeweils 30µl diverser Beadvarianten resuspendiert. Nach Übertragung in 96well Kavitäten wurde das Prälysat durch Zentrifugation durch eine einschichtige Glasmatrix bewegt.

Nach einem ethanolischen Waschschritt, erfolgte die Elution mit 30µl TE Puffer. Fig. 4 zeigt die gelelektrophorethische Analyse der Präparationen im Vergleich zu einer herkömmlichen Plasmid Mini Präparation mit dem Perfect Prep Plasmid Mini Kit der Firma Eppendorf. Der Vergleich zeigt, daß prinzipiell unterschiedliche Beads verwendet werden können, diese jedoch gemäß ihrer Materialeigenschaften zu Ausbeutedifferenzen führen. Verwendete Beads, Spuren: **(1)** Davisil 200-425, Silica Porengröße 150 Angstroem, Partikelgröße 33-75 µm, **(2)** SephadexG200, Dextran quervernetzt mit Epichlorhydrin, Partikelgröße 25-100 µm, **(3)** Sephadex LH20, Partikelgröße 20-100, **(4)** CelatomFW14, Cellulose, **(5)** Celatom FW50, Cellulose, **(6)** Celatom FW60, Cellulose, **(7)** CelatomFW80, Cellulose, **(8)** AFR25PDB-SO₃, **(9)** CK10M/4PDB-SO0₃, Polystyrol quervernetzt mit Divinylbenzol, Ionenaustauscher, Partikelgröße ca. 20 µm, **(10)** POROSR1-10, Polystyrol quervemetzt mit Divinylbenzol, **(11)** POROSR1-20, Polystyrol quervernetzt mit Divinylbenzol, **(12)** POROSR2-10, Polystyrol quervernetzt mit Divinybenzol, **(13)** POROSR2-20, Polystyrol quervernetzt mit Divinylbenzol Kontrollen: **(14)** Glasbeads, **(15)** ohne Beads, **(16)** leer, **(17)** Perfect Prep Mini.

### Beispiel 4: Isolierung von Plasmiden unter Einsatz unterschiedlicher Filtermatrizes.

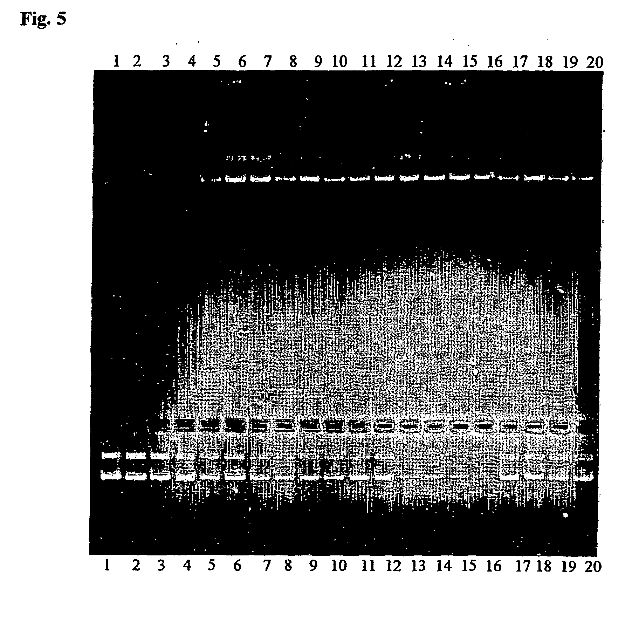

Die Zellen wurden jeweils in LB Medium angezogen und dann pelletiert. Die Zell-Pellets wurden jeweils in 400 µl Puffer I + 30 µl Glasbeads aufgenommen und die erhaltene Suspension mittels Vacuum oder Zentrifugation durch einschichtige und zweischichtige Filtermatrices in 96well Platten gedrückt. Nach ethanolischem Waschen, wurde dann mit jeweils 30 µl TE-Puffer eluiert und die Eluate wurden gel-elektrophoretisch analysiert (Fig. 5).

Man erkennt, daß das erfindungsgemäße Verfahren unter Verwendung unterschiedlicher Matrices einen guten Präparationserfolg liefert. Die Zuordnung der Spuren zu den Filtermatrizes (jeweils 4 Parallelen) ist wie folgt:
Spuren oben:
   Spuren 1-4 = PVDF 0,45µm; Spuren 5-8 = Glasfaser/C; Spuren 9-12 = Glasfaser/F; Spuren 13-16 = oberflächenmodifiziertes Nylon; Spuren 17-20 = Glasfaser/F+MBD;
Spuren unten:
   Spuren 1-4 = PVDF 0,5µm; Spuren 5-8 = PKB(B); Spuren 9-12 = Nylon 0,4µm; Spuren 13-16 = Glasfaser X; Spuren 17-20 = Glasfaser/C 2-lagig.

### Beispiel 5: Isolierung von Plasmiden, wobei die Zellen in einem ersten Schritt vollständig lysiert werden.

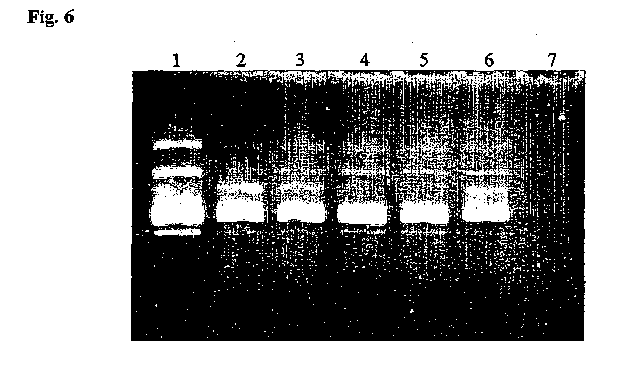

Die Zellen wurden jeweils in 1,2 ml LB Medium angezogen und dann pelletiert. Die Zell-Pellets wurden jeweils in 400 µl Puffer I + 30 µl Beads (Prälyse) bzw. in Puffer IV (Lyse) aufgenommen und die erhaltene Suspension mittels Zentrifugation durch eine einschichtige Filtermatrix in Einzelsäulen gedrückt (A) bzw. die Suspension auf dem Vorwege Pelletiert (B). Nach ethanolischem Waschen, wurde dann mit jeweils 30 µl TE-Puffer eluiert und die Eluate wurden gel-elektrophoretisch analysiert. Das Ergebnis zeigt Fig. 6. **Kontrolle** (Spur 1) Perfect Prep Mini, **Vollyse:** (Spuren 2+3) lytische Bedingungen (Puffer IV + 30 µl Beads) + Filtermatrix wie (A), (Spuren 4+5) lytische Bedigungen (Puffer IV + Beads) ohne Filtermatrix, Zelltrümmer wurden abzentrifugiert und dann der Überstand erneut über eine Filtermatrix präpariert wie (B). **Prälyse:**(Spur 6) Puffer I + Beads über Filtermatrix wie (A), (Spur 7) Puffer I + Beads, nach Zentrifugation wurde nur der Überstand wie unter (Spuren 3+4) bzw.(B) über eine Filtermatrix präparariert. Die Ergebnisse zeigen, daß die Verwendung unterschiedlicher Puffer die gezielte Einstellung einer vollständigen Lyse bzw. einer prälytischen Zellfragilisierung erlaubt, wobei letztere mechanische Scherkräfte für die Vollyse benötigt.

### Beispiel 6: Folgapplikation - Sequenzierqualität der Plasmid DNA

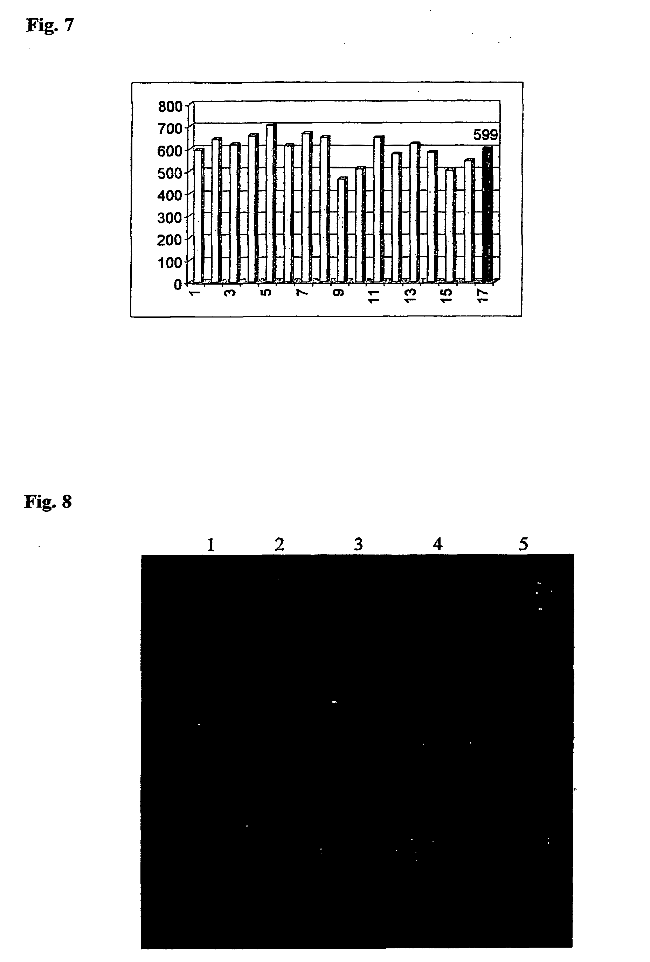

Bakterien wurden jeweils in 1,2 LB Medium angezogen und dann pelletiert. Die Zell-Pellets wurden jeweils in 400 µl Puffer I + 30 µl Glasbeads aufgenommen und die erhaltene Suspension mittels Zentrifugation durch eine einschichtige Glasfiltermatrix gedrückt. Nach ethanolischem Waschen wurde die Plasmid DNA mit 30 µl TE-Puffer eluiert und Aliquots von je 4 µl mittels automatischer Kapillarsequenzierung analysiert und die phred20 Scores ermittelt (Fig. 7). Die Ergebnisse zeigen, daß die Plasmid DNA Präparation nach dem anmeldungsgemäßen Verfahren eine gute Sequenzierung erlaubt, identisch zu einer hochreinen Kontrolle (siehe Balken 17).

### Beispiel 7: Lyse unter hypoosmotischen Bedingungen

Bakterien wurden jeweils in 1,2 LB Medium angezogen und dann pelletiert. Die Zell-Pellets wurden jeweils in 400 µl Puffer I und Puffer II + 30 µl Glasbeads aufgenommen und die erhaltene Suspension mittels Zentrifugation durch eine einschichtige Glasfiltermatrix gedrückt. Nach ethanolischem Waschen wurde die Plasmid DNA mit 30 µl TE-Puffer eluiert und das Eluat wurde gelelektrophoretisch analysiert (Fig. 8; Spur 1: MiniPrep; Spur 2:Puffer I; Spur 3: Puffer II; Spur 4: MiniPrep; Spur 5: Puffer II). Es zeigte sich, daß die Bedingungen für eine Prälyse mit nachfolgender Lyse an der Matrix sowohl unter Verwendung einer hypoosmotischen, als auch einer hyper-osmotischen Pufferzusammensetzung erzielt werden kann.

## Patentansprüche

1. Verfahren zur Isolierung von Plasmiden aus Bakterienzellen, bei dem
a) eine Zellsuspension hergestellt wird,
b) die suspendierten Zellen lysiert,
c) mit einem Fällungsmittel kontaktiert werden, das in der Lage ist, die Konformation freigesetzter Plasmide zu verändern, und
d) durch eine Filtermatrix bewegt werden, wobei die freigesetzten Plasmide in bzw. an der Filtermatrix zurückgehalten werden,
e) gegebenfalls die Filtermatrix mit einer Waschlösung gewaschen wird, und
f) die zurückgehaltenen Plasmide selektiv aus der Filtermatrix eluiert werden, **dadurch gekennzeichnet, dass**
gemeinsam die Suspension der Zellen (a), die Zelllyse (b) und die Kontaktierung mit einem Fällungsmittel (c) durch Zugabe eines einzigen Puffers bewirkt werden.

2. Verfahren nach Anspruch 1, **dadurch gekennzeichnet, dass** die Zugabe des Puffers eine vollständige Lyse der Zellen bewirkt.

3. Verfahren nach Anspruch 1, **dadurch gekennzeichnet, dass** die Zugabe des Puffers eine Vorschädigung der Zellen bewirkt, und eine vollständige Lyse der Zellen erst bei Kontakt mit der Filtermatrix erfolgt.

4. Verfahren nach einem der Ansprüche 1 bis 3, **dadurch gekennzeichnet, dass** als Fällungsmittel Polyethylenglykol (PEG) eingesetzt wird.

5. Verfahren nach Anspruch 3, **dadurch gekennzeichnet, dass** der Puffer als Vorschädigung der Zellen hervorrufende Substanzen Enzyme und/oder milde Detergenzien und/oder osmotisch wirksame Substanzen enthält, insbesondere Salze oder Zucker, die die Zellen unter osmotischen Streß setzen.

6. Verfahren nach einem der Ansprüche 1 bis 5, **dadurch gekennzeichnet, dass** das Verfahren mit einer Filtermatrix arbeitet, die zwei Filterschichten aufweist.

7. Verfahren nach einem der Ansprüche 1 bis 6, **dadurch gekennzeichnet, dass** Beads zugesetzt werden, die sich im Zuge, insbesondere infolge eines Zentrifugations- oder Filterschritts zur Filtermatrix bzw. zur oberen Schicht einer Filtermatrix anordnen.

8. Verfahren nach Anspruch 7, **dadurch gekennzeichnet, dass** die Beads gemeinsam mit der mindestens einen konformationsändernden und/oder der mindestens einen lysierend wirkenden Substanz in einem Puffer enthalten sind.

9. Verfahren nach einem der Ansprüche 1 bis 8, **dadurch gekennzeichnet, dass** zur Elution der Plasmide von der Filtermatrix ein Puffer eingesetzt wird, der die Konformationsänderung der zu isolierenden Plasmide wieder rückgängig macht.

## Claims

1. A method for isolating plasmids from bacterial cells, wherein
a) a suspension of cells is prepared,
b) the suspended cells are lysed,
c) are brought into contact with a precipitating agent able to induce a change in conformaton of the released plasmids, and
d) are moved through a filter matrix with the released plasmids being retained in or at this filter matrix,
e) where called for, the filter matrix is washed with a washing solution and
f) the retained plasmids are selectively eluted from the filter matrix,
**characterized in that** the suspension of the cells (a), the lysis of the cells (b) and the bringing into contact with a precipitating agent (c) are effected together by addition of one single buffer.

2. Method as claimed in claim 1, **characterized in that** the addition of the buffer effects complete cell lysis.

3. Method as claimed in claim 1, **characterized in that** the addition of the buffer effects a pre-damaging of the cells and that complete lysis occurs only upon contact of the cells with the filter matrix.

4. Method as claimed in one of claims 1 through 3, **characterized in that** the precipitating agent is polyethyleneglycol (PEG).

5. Method as claimed in claim 3, **characterized in that** the buffer includes as substances causing pre-damaging of the cells, enzymes and/or mild detergents and/or osmotic substances, in particular salts or sugars, causing osmotic stress to the cells.

6. Method as claimed in one of claims 1 through 5, **characterized in that** the method employs a filter matrix comprising two filter layers.

7. Method as claimed in one of claims 1 through 6, **characterized by** adding beads which, in the course of processing, in particular as a consequence of a centrifuging or a filter stage, will form the filter matrix or will collect as the upper layer of a filter matrix.

8. Method as claimed in claim 7, **characterized in that** the beads are contained in one buffer together with the at least one conformation-altering and/or the at least one lysing substance.

9. Method as claimed in one of claims 1 through 8, **characterized in that** a buffer is used to elute the plasmids from the filter matrix which reverses the conformation-alteration of the plasmids to be isolated.

## Revendications

1. Procédé d'isolement de plasmides de cellules bactériennes consistant à
a) préparer une suspension de cellules,
b) lyser les cellules en suspension,
c) les mettre en contact avec un précipitant capable de modifier la conformation des plasmides libérés, et
d) les faire passer à travers une matrice filtrante, les plasmides libérés étant retenus dans la matrice ou à la surface de celle-ci,
e) laver éventuellement la matrice filtrante avec une solution de lavage, et
f) éluer sélectivement les plasmides retenus dans la matrice filtrante, **caractérisé en ce que**
la suspension des cellules (a), la lyse des cellules (b) et la mise en contact avec un précipitant (c) sont réalisées par adjonction d'une solution tampon unique.

2. Procédé selon la revendication 1, **caractérisé en ce que** l'adjonction de la solution tampon entraîne une lyse complète des cellules.

3. Procédé selon la revendication 1, **caractérisé en ce que** l'adjonction de la solution tampon entraîne une dégradation préliminaire des cellules et que la lyse complète des cellules n'a lieu qu'au contact avec la matrice filtrante.

4. Procédé selon l'une des revendications 1 à 3, **caractérisé en ce que** le précipitant mis en oeuvre est du polyéthylèneglycol (PEG).

5. Procédé selon la revendication 3, **caractérisé en ce que** la solution tampon contient des enzymes comme substances entraînant une dégradation préliminaire des cellules et/ou des détergents doux et/ou des substances a effet osmotique, notamment des sels ou du sucre, qui exposent les cellules à des contraintes osmotiques.

6. Procédé selon l'une des revendications 1 à 5, **caractérisé en ce que** le procédé met en oeuvre une matrice filtrante qui présente deux couches filtrantes.

7. Procédé selon l'une des revendications 1 à 6, **caractérisé en ce que** l'on ajoute des billes qui, lors d'une étape de centrifugation, et notamment du fait de celle-ci, ou lors d'une étape de filtrage s'agencent pour former une matrice filtrante ou la couche supérieure d'une matrice filtrante.

8. Procédé selon la revendication 7, **caractérisé en ce que** les billes sont contenues dans une solution tampon avec la substance entraînant au moins une modification de conformation et/ou la substance ayant au moins un effet de lyse.

9. Procédé selon l'une des revendications 1 à 8, **caractérisé en ce que** la solution mise en oeuvre pour éluer les plasmides de la matrice filtrante est une solution tampon qui entraîne la réversion de la modification de conformation des plasmides à isoler.
